# EUROPEAN PATENT SPECIFICATION

(11) **EP 2 548 587 B1**
(45) Date of publication and mention of the grant of the patent: **22.02.2017**
(21) Application number: 12176947.5
(22) Date of filing: 18.07.2012
(51) Int. Cl.: A61L 27/16, A61L 27/30, A61L 27/50, A61L 31/04, C08L 27/18

(54) **Coated implant and method for its production**
Beschichtetes Implantat und Verfahren zu dessen Herstellung
Implant revêtu et son procédé de production

(30) Priority: 22.07.2011 DE 102011079680
(43) Date of publication of application: 23.01.2013
(73) Proprietor: Aesculap AG, 78532 Tuttlingen/Donau (DE)
(72) Inventor: Goldman, Helmut, 34119 Kassel (DE); Langanke, Dennis, 72074 Tübingen (DE)
(74) Representative: Patentanwälte Ruff, Wilhelm, Beier, Dauster & Partner mbB

(56) References cited:
- EP-A1- 0 633 032
- EP-A1- 2 359 876
- US-A- 5 061 276
- US-A1- 2006 051 544
- WOODYARD L L ET AL: "A comparison of the effects of several silver-treated intravenous catheters on the survival of staphylococci in suspension and their adhesion to the catheter surface", JOURNAL OF CONTROLLED RELEASE, ELSEVIER, AMSTERDAM, NL, vol. 40, no. 1, 1 June 1996 (1996-06-01), pages 23-30, XP004037348, ISSN: 0168-3659, DOI: 10.1016/0168-3659(95)00135-2

## Description

The invention relates to a coated implant having a node-fibril structure and to a method for its production.

Vascular prosthesis infections in reconstructive vascular surgery often entail serious complications. Despite routinely applied systematic antibiotic prophylaxis and high medical treatment standards, prosthesis infections result in high amputation and mortality rates.

This applies particularly to infections after implantation of non-textile vascular prostheses such as, for example, ePTFE vascular prostheses. As will be explained in more detail below, this is connected in particular with the fact that antimicrobial additivation of such vascular prostheses, with the aim of long-term protection against infections, proves very difficult and is furthermore associated with risks to the patient.

Vascular prostheses made of expanded polytetrafluoroethylene (ePTFE) have been known for a long time and are used, owing to their antithrombotic and biocompatible properties, above all to replace small-lumen vessels. Corresponding prostheses are described for example in the documents US 4,208,745; US 4,713,070 and US 4,877,661.

Vascular prostheses made of ePTFE have a microstructure in which nodes are connected to one another via fibrils (node-fibril structure). This microstructure is the result of a production process based on a shaping step, generally extrusion, and a subsequent stretching step.

US 5,433,909 discloses a vascular prosthesis made of ePTFE having a pore size gradient, the pore size increasing from the inside to the outside of the prosthesis. The larger pores on the outside on the one hand fundamentally improve the connective tissue growth into the prosthesis and therefore the incorporation thereof into the surrounding tissue.

On the other hand, larger pores in an ePTFE microstructure generally correspond to a greater mutual spacing of the nodes (internodal space) and therefore longer and in particular thinner fibrils.

A thinner configuration of the fibrils, however, fundamentally makes it more difficult to coat the prosthesis since the risk is thereby increased that the fibrils and therefore the node-fibril structure overall will be damaged. This applies above all in respect of the use of high-energy coating methods such as, for example, the IBAD method (Ion Beam Assisted Deposition). A corresponding coating method is described, for example in DE 103 23 676 A1.

US 5,061,276 discloses a vascular graft made of PTFE having a node-fibril structure, wherein the graft is coated with an elastomeric polymer by spraying, dipping or impregnating the surface of the graft before the step of stretching the graft.

EP 2 359 876 A1 discloses a vascular graft implant made of PTFE having a node-fibril structure, wherein an antimicrobial coating is applied to the surface of the implant, preferably using the IBAD method.

Woodyard et al. (Journal of Controlled Release 40 (1996) 23-30) discloses IBAD silver treated catheters.

If implants having a node-fibril structure are subjected to ion bombardment, they may be "broken up" in such a way that individual fibrils remain connected to nodes only on one side. In relation to such fibrils, however, the risk of full detachment arises. As a result of this, these fibrils may enter the surrounding tissue and lead to serious inflammatory reactions.

It is therefore an object of the present invention to provide a coated implant having a node-fibril structure, which overcomes difficulties known from the prior art. In particular, the implant should have maximally long-term antimicrobial properties owing to its coating and preferably have an at least substantially intact node-fibril structure in spite of its coating.

This object is achieved in terms of method technology by a method having the features of independent Claim 1. Preferred embodiments of the method are the subject of dependent Claims 2 to 7

In terms of product technology, the object of the invention is achieved by an implant having the features according to Claim 8. Preferred embodiments of the implant protected in Claim 8 are presented in dependent Claims 9 to 14.

The method according to the invention is a method for producing a coated, in particular partially coated implant, comprising the following steps:
a)coating an unstretched implant preform, comprising polytetrafluoroethylene and/or a copolymer comprising tetrafluoroethylene as a monomer unit, with an antimicrobially effective metal, wherein the coating is carried out by means of ion beam assisted deposition (IBAD) and
b)stretching the coated implant preform.

The method according to the invention is therefore a method for producing an antimicrobially effective implant.

In the method according to the invention, the coating is so to speak "prelaid" by coating an implant preform, which has not yet been stretched, with an antimicrobially effective metal. The stretching does not take place until after the coating.

Owing to the compact structure of the as yet unstretched implant preform, it is possible to use ion beam assisted deposition (IBAD), without thereby compromising or substantially compromising a node-fibril structure which is only produced in the subsequent stretching step.

An implant preform in the context of the present invention is intended to mean an implant precursor for the production of the implant according to the invention.

An unstretched implant preform in the context of the present invention is intended to mean an implant precursor which does not yet have a node-fibril structure. In accordance with this, the polytetrafluoroethylene and/or the copolymer comprising tetrafluoroethylene as a monomer unit does not yet form a node-fibril structure in the unstretched implant preform.

Stretching, also referred to as expansion, in the context of the present invention is intended to mean the stretching or expanding of an implant preform, generally while heating it, as a result of which the implant preform is drawn apart or elongated so as to form a node-fibril structure.

A copolymer in the context of the present invention is intended to mean a polymer which comprises two or more different types of monomer units.

Besides tetrafluoroethylene, a copolymer according to the present invention preferably comprises at least one further monomer unit (so-called comonomer unit) which is selected from the group consisting of chlorotrifluoroethylene, perfluoroalkoxytetrafluoroethylene, hexafluoropropylene, and tetrafluoropropylene.

An antimicrobially effective or antimicrobial metal in the context of the present invention is intended to mean a metal which reduces or inhibits the growth, multiplication and/or infectiosity of microorganisms, for example bacteria, viruses, fungi and the like, or kills or inactivates microorganisms.

In a preferred embodiment, the implant preform consists of polytetrafluoroethylene and/or a copolymer comprising tetrafluoroethylene as a monomer unit. If required, however, the polytetrafluoroethylene and/or the copolymer may also be provided with additives or auxiliaries.

In a preferred embodiment, the implant preform is an unsintered implant preform.

The implant preform may, in particular, be a cavity implant preform, preferably a tubular or cylindrical cavity implant preform.

In another embodiment, the implant preform is formed as a cavity implant preform having an inner diameter of between 4 and 40 mm, in particular between 4 and 20 mm, preferably between 4 and 12 mm. For the production of a small-lumen cavity implant, it may be preferable to use a cavity implant preform having an inner diameter ≤ 6 mm, in particular between 4 and 6 mm. With respect to possible configurations of the cavity implant preform, reference is made to the embodiments below.

In a further embodiment, the implant preform is intended to replace hollow organs.

The implant preform is preferably a prosthesis preform, in particular a vascular prosthesis preform, preferably an arterial vascular prosthesis preform.

Correspondingly, the implant to be produced may be a cavity implant, preferably a prosthesis, in particular a vascular prosthesis, preferably an arterial vascular prosthesis.

A vascular prosthesis in the context of the present invention is intended to mean a prosthesis for the replacement of natural blood vessels, in contrast to so-called endoluminal prostheses such as in particular stents.

In an alternative embodiment, the implant to be produced may be a covering or sheath (graft), in particular a covering or sheath for a stent graft. The covering or sheath in this embodiment is expediently formed as a tube or cylinder.

In another alternative embodiment, the implant preform is in the form of a two-dimensional implant preform without a cavity or lumen, for example as a patch or the like. Correspondingly, according to the invention the implant to be produced may be a two-dimensional implant such as, for example, a wound patch or the like.

The implant preform is coated with an antimicrobially effective metal. The metal is preferably selected from the group consisting of iridium, aluminium, zinc, copper, silver, gold, titanium, zirconium, platinum, palladium and combinations, in particular alloys, thereof.

For the coating, in an expedient embodiment, the implant preform is applied onto a support, in particular onto a rotationally symmetric and preferably rotatable support. The support is preferably formed as a tube or cylinder. For example, the support may be formed as a mandrel, spindle, pin, shaft or the like. The support itself may furthermore consist of metal, steel or a plastic.

In another expedient embodiment, the implant preform is transferred into a vacuum chamber or reduced-pressure chamber before the coating.

The implant preform is coated by means of Ion Beam Assisted Deposition (IBAD).

In the physical method mentioned above, a starting metal is converted into the gas phase. The gaseous metal is subsequently conveyed to a substrate to be coated, where it condenses and leads to the formation of an intended layer. The metal to be deposited is provided in solid form in a usually evacuated coating chamber. By bombardment with laser beams, ions or electrons and by arc discharge, the metal, which is often also referred to as a target, is evaporated. The evaporated metal then moves either ballistically or propelled by electric fields through the chamber and thereby strikes the substrate to be coated, where the layer formation takes place. So that the evaporated metal particles can actually reach the substrate to be coated and are not lost for instance by scattering from the gas particles, operation is generally carried out at a reduced pressure. In order to achieve a maximally homogeneous coating of the substrate, it may be moved in a suitable way, in particular rotated, during the coating process.

The ion beam assisted deposition (IBAD) mentioned above is known per se to the person skilled in the art, so that more extensive comments will be omitted here.

The ion beam assisted deposition may be carried out at a reduced pressure of at least 10⁻⁴ Torr, in particular at least 10⁻⁵ Torr. It is furthermore preferable for the ion beam assisted deposition to be carried out at a temperature above 150°C. It is furthermore preferable to operate with an ion beam energy of between 5 and 10 000 eV, in particular between 50 and 5000 eV, preferably between 200 and 1000 eV. The current density generated on the surface of the implant preform during the ion beam assisted deposition may lie between 0.1 and 500 µA/cm², in particular between 1 and 250 µA/cm², preferably between 10 and 80 µA/cm². The deposition of the material may be carried out within a time period of between 0.1 and 500 minutes, in particular between 1 and 200 minutes, preferably between 5 and 75 minutes. The deposition of the material during the ion beam assisted deposition is preferably carried out with a deposition rate of between 0.1 and 50 Å/s, in particular between 1 and 25 Å/s, preferably between 2 and 10 Å/s.

In a particularly preferred embodiment, the implant preform is coated with silver by means of or in the scope of ion beam assisted deposition (IBAD).

Depending on the coating metal used, the coated but as yet unstretched implant preform may have a metallically, in particular silvery lustrous surface, in particular outer surface.

As already mentioned in the introduction, according to the invention the implant preform is not stretched until after the coating.

In a preferred embodiment, the coated implant preform is stretched to from 0.5 to 10 times, in particular 1.5 to 7 times, preferably 3 to 5 times its original length, i.e. its length before stretching and in particular before coating.

The coated implant preform is preferably stretched in the heated state, for example in a temperature range of between 150 and 300°C, in particular between 170 and 250°C, preferably between 190 and 230°C.

In another preferred embodiment, the implant preform is subjected to a sintering process after the stretching. The sintering particularly advantageously leads to strengthening and therefore stabilization of the node-fibril structure obtained as a result of the stretching.

The stretched implant preform is preferably sintered above the crystallization temperature of polytetrafluoroethylene and/or of the copolymer comprising tetrafluoroethylene as a monomer unit.

In general, the stretched implant preform is sintered at a temperature above 327°C, in particular at a temperature of between 328 and 360°C, preferably at a temperature of 350°C.

In another embodiment, the stretched implant preform is sintered for a time period of from 5 to 30 min, in particular 5 to 20 min, preferably 5 to 15 min.

If the coated implant preform is stretched in the heated state, it may be expedient to cool the implant preform after the stretching and before a sintering step.

After the stretching, in particular after a sintering step, depending on the coating metal used, the implant preform may have a slightly or matt grey surface, in particular outer surface.

A second aspect of the present invention relates to an implant, in particular a prosthesis, having a node-fibril structure, which is produced or producible by the method according to the invention.

The implant is preferably formed from expanded polytetrafluoroethylene and/or an expanded copolymer comprising tetrafluoroethylene as a monomer unit.

In an advantageous embodiment, the node-fibril structure is an intact i.e. undamaged node-fibril structure. An intact node-fibril structure in the context of the present invention is preferably characterized by the following embodiment.

In a further embodiment, all the fibrils of the node-fibril structure are connected to nodes on both sides, i.e. via both fibril ends. In other words, it is advantageous for the node-fibril structure to be free of fibrils which are connected to nodes on only one side, i.e. via only one fibril end. This has the advantage that undesired detachment of fibrils, as described in the introduction, can be avoided.

Nodes, in particular all the nodes, of the node-fibril structure are preferably coated with the antimicrobially effective metal over a depth of from 1 to 150 µm, in particular 10 to 100 µm, preferably 20 to 40 µm, measured from the surface of the implant, in particular the outer surface.

In another embodiment, the fibrils of the node-fibril structure may be uncoated, i.e. free of coating with the antimicrobially effective metal.

As an alternative, fibrils of the node-fibril structure, generally fibrils occurring on the surface of the implant, in particular the outer surface, may be at least partially coated, and in particular only partially coated. Here, partially coated fibrils are intended to mean fibrils which are not coated fully, i.e. not coated surface-wide or continuously, with the antimicrobially effective metal.

According to a possible embodiment, fibrils occurring on the surface of the implant, in particular the outer surface, may be fully coated with the antimicrobially effective metal.

In another embodiment, the implant comprises a proportion of the antimicrobially effective metal between 0.01 and 5.0 wt%, in particular between 0.05 and 3.0 wt%, preferably between 0.1 and 1.5 wt%, expressed in terms of the total weight of the implant.

In another embodiment, the coated nodes have a layer thickness of the antimicrobially effective metal between 50 and 4000 Å, in particular between 200 and 2000 Å, preferably between 250 and 1500 Å.

The fibrils of the node-fibril structure may have a length of between 1 and 150 µm, in particular between 10 and 100 µm, preferably between 20 and 80 µm.

The fibrils may furthermore have a diameter of between 0.1 and 10 µm, in particular between 0.5 and 5 µm.

The distance between the nodes, i.e. the so-called internodal space, in the node-fibril structure may furthermore lie between 1 and 150 µm, in particular between 10 and 100 µm, preferably between 20 and 80 µm.

The internodal spaces are preferably free of theantimicrobially effective metal. In this way, it is particularly advantageously possible for connective tissue cells to grow in, so that the anchoring of the implant in the body of a patent is additionally improved. In other words, according to the invention it is preferable for the internodal spaces of the node-fibril structure not to be covered or coated with the antimicrobially effective metal.

In order to additionally promote the inward growth of connective tissue cells, according to the invention the implant may furthermore be free of impregnation coating, and in particular impregnation coating which covers and/or fills the internodal spaces.

The nodes of the node-fibril structure usually have an elongate, in particular ellipsoidal shape. The nodes are preferably distinguished by an essentially, in particular completely, uniform shape and in particular size. The node size, in particular node length, may lie between 1 and 800 µm, in particular between 5 and 500 µm, preferably between 10 and 300 µm.

The fibrils connecting the nodes may be formed in a curved, undulating and/or straight shape.

In another embodiment, the implant is formed as a cavity replacement implant, in particular as a vascular prosthesis, preferably as an arterial vascular prosthesis.

In another embodiment, the implant is a cavity replacement implant having an inner diameter of between 4 and 40 mm, in particular between 4 and 20 mm, preferably between 4 and 12 mm. If the implant is a small-lumen cavity replacement implant, inner diameters ≤ 6 mm, in particular between 4 and 6 mm, may be preferred. With respect to possible configurations of the cavity replacement implant, full reference is made to the previous embodiment.

In an alternative embodiment, the implant is in the form of a two-dimensional implant without a cavity or lumen, for example as a wound patch or the like.

The antimicrobially effective metal is preferably selected from the group consisting of iridium, aluminium, zinc, copper, silver, gold, titanium, zirconium, platinum, palladium and combinations, in particular alloys, thereof.

In an expedient embodiment, the implant is sterilized and in particular packaged.

With respect to other features and advantages of the implant, full reference is made to the entire description.

At this point, the advantages of the present invention will be summarized once more as follows:
- The invention is based on the concept of coating an implant preform, which has not yet been stretched, with an antimicrobially effective metal such as for example silver, before subsequently stretching it in order to produce a node-fibril structure. The "prelaying" of the coating has the advantage that owing to its more compact structure, the unstretched implant preform can be coated by means of the IBAD method.
- Because the stretching does not take place until after the coating of the implant preform has been carried out, damage to the node-fibril structure existing after the stretching can be at least substantially avoided.
- This in turn has the advantage that large stretching factors can also be used, which lead to large node-node spacings and therefore to very thin fibrils, without thereby substantially compromising the intactness of the node-fibril structure.

- Furthermore, in the scope of the present invention, it has surprisingly been found that implant preforms having thin antimicrobially effective metal layers can also readily be stretched with the desired stretching factors.
- Depending on the type of antimicrobially effective metal, various properties, and optionally multifunctional properties, can be imparted to the implant to be produced. In this case it has been found that even small layer thicknesses are sufficient for medium- to long-term antimicrobial protection. Small layer thicknesses of antimicrobially effective metals can be advantageous, particularly for small-lumen vascular prostheses, with a view to maintaining their structural integrity.

Further details, features and advantages of the invention may be found in the following description of preferred embodiments with the aid of figures, a production example and the dependent claims. In these embodiments, individual features of the invention may be implemented separately or in combination with other features. The preferred embodiments described are merely to be understood as descriptive and entirely nonlimiting disclosure. All the figures are hereby incorporated into the content of this description by explicit reference.

In the figures:
Figure 1 shows the SEM image of a vascular prosthesis according to the invention,
Figure 2 shows the SEM image of a vascular prosthesis known from the prior art, made of ePTFE,
Figure 3 shows the photographic image of a prosthesis preform coated with silver but not yet stretched,
Figure 4 shows the photographic image of the prosthesis preform represented in Figure 3 after its stretching.

### Description of the Figures

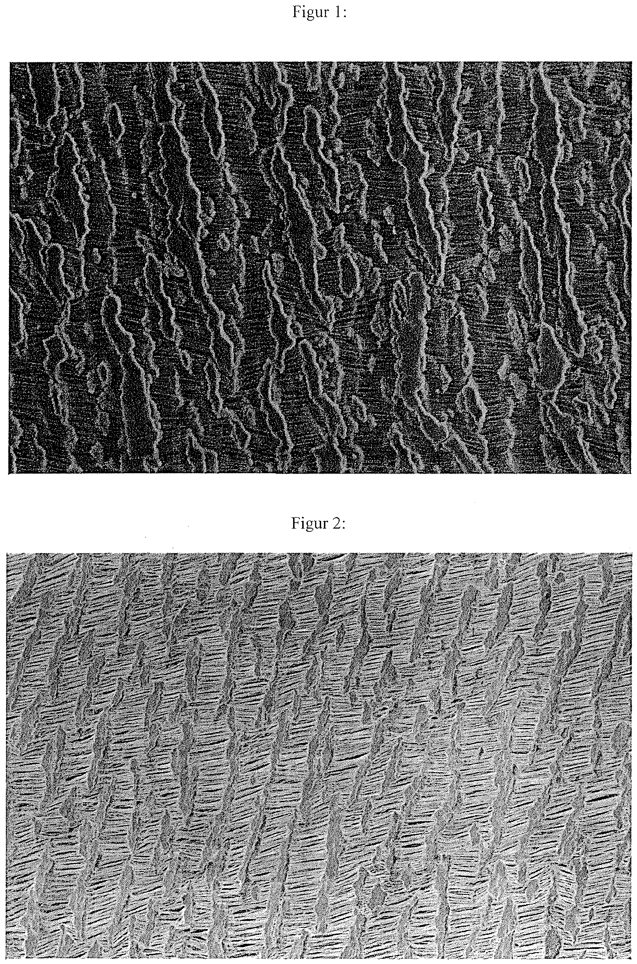

Figure 1 shows the SEM image of a vascular prosthesis made of ePTFE, which is partially coated with elemental silver on its outer surface. In order to produce the prosthesis, the silver was applied by means of the IBAD method onto a PTFE prosthesis preform which had not yet been stretched. Stretching of the coated prosthesis preform was subsequently carried out to form the ePTFE vascular prosthesis represented in Figure 1.

The internodal spaces of the node-fibril structure are free of silver coating.

Figure 1 shows an intact, or structurally unimpaired, node-fibril structure in which the nodes are connected to one another via fibrils. The node-fibril structure, represented in Figure 1, of the coated vascular prosthesis is comparable to the node-fibril structure of a conventional uncoated ePTFE vascular prosthesis (Figure 2).

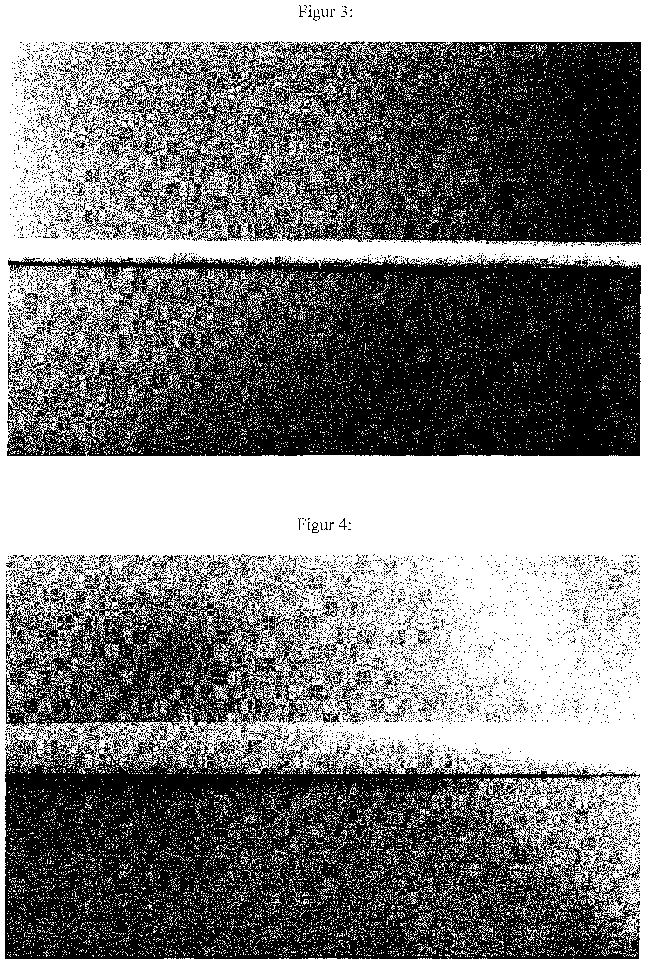

Figure 3 shows the photographic image of a PTFE prosthesis preform after it has been coated with silver. The outer surface of the coated prosthesis preform is silvery lustrous. After the stretching, however, the outer surface has a matt grey appearance (Figure 4).

### Production Example

An unstretched prosthesis preform made of polytetrafluoroethylene, having an inner diameter of 6 mm, was fitted over a rotatable metal pin having an outer diameter < 6 mm and fastened horizontally in a vacuum chamber. After closing the vacuum chamber and applying a vacuum of 10⁻⁴ Torr, the prosthesis preform was coated with silver by means of ion beam assisted deposition (IBAD). The coating process was terminated after reaching a silver layer thickness of 1000 Å. The prosthesis preform was thereupon removed from the vacuum chamber.

The prosthesis preform coated in this way was subsequently connected on both sides to a stretching device and heated in a heating oven for 15 minutes at a temperature of 210°C. The prosthesis preform was stretched to four times its length by simultaneous and uniform pulling in the heated state.

The preform stretched in this way was cooled in the fixed state and subsequently sintered for 10 minutes in a sintering oven.

The ePTFE vascular prosthesis produced in this way had an inner diameter of 6 mm, a wall thickness of 0.6 mm and a matt grey outer surface. On its outer surface, the vascular prosthesis had a silver coating with a proportion of 0.3 wt%, expressed in terms of the total weight of the prosthesis. The silver coating furthermore had a penetration depth of from 20 to 40 µm.

## Claims

1. Method for producing a coated implant, comprising the steps:
a) coating an unstretched implant preform, comprising polytetrafluoroethylene and/or a copolymer comprising tetrafluoroethylene as a monomer unit, with an antimicrobially effective metal, wherein the coating is carried out by means of ion beam assisted deposition (IBAD) and
b) stretching the coated implant preform.

2. Method according to claim 1, **characterized in that** the implant preform is an unsintered preform.

3. Method according to claim 1 or 2, **characterized in that** the implant preform is a prosthesis preform, preferably a vascular prosthesis preform.

4. Method according to one of the preceding claims, **characterized in that** the antimicrobially effective metal is selected from the group consisting of iridium, aluminium, zinc, copper, silver, gold, titanium, zirconium, platinum, palladium and combinations, in particular alloys, thereof.

5. Method according to one of the preceding claims, **characterized in that** the implant preform is coated with silver.

6. Method according to one of the preceding claims, **characterized in that** the coated implant preform is stretched to from 0.5 to 10 times, in particular 1.5 to 7 times, preferably 3 to 5 times its original length.

7. Method according to one of the preceding claims, **characterized in that** the coated implant preform is sintered after the stretching, preferably above the crystallization temperature of polytetrafluoroethylene and/or of the copolymer comprising tetrafluoroethylene as a monomer unit.

8. Implant having a node-fibril structure produced or producible by a method according to one of the preceding claims.

9. Implant according to claim 8, **characterized in that** all the fibrils of the node-fibril structure are connected on both sides to the nodes.

10. Implant according to claim 8 or 9, **characterized in that** the fibrils of the node-fibril structure are free of coating with the antimicrobially effective metal.

11. Implant according to claim 8 or 9, **characterized in that** fibrils occurring on the surface of the implant, in particular the outer surface, are at least partially coated, and preferably only partially coated.

12. Implant according to one of claims 8 to 11, **characterized in that** the implant comprises a proportion of the antimicrobially effective metal between 0.01 and 5.0 wt%, in particular between 0.05 and 3.0 wt%, preferably between 0.1 and 1.5 wt%, expressed in terms of the total weight of the implant.

13. Implant according to one of claims 8 to 12, **characterized in that** the coated nodes have a layer thickness of the antimicrobially effective metal between 50 and 4000 Å, in particular between 200 and 2000 Å, preferably between 250 and 1500 Å.

14. Implant according to one of claims 9 to 13, **characterized in that** the internodal spaces of the node-fibril structure are free of coating with the antimicrobially effective metal.

## Patentansprüche

1. Verfahren zur Herstellung eines beschichteten Implantats, umfassend die Schritte:
a) Beschichten eines unverstreckten Implantatrohlings, umfassend Polytetrafluorethylen und/oder ein Tetrafluorethylen als Monomereinheit umfassendes Copolymer, mit einem antimikrobiell wirksamen Metall, wobei die Beschichtung mittels ionenstrahlgestützter Deposition bzw. Abscheidung (IBAD) durchgeführt wird, und
b) Verstrecken des beschichteten Implantatrohlings.

2. Verfahren nach Anspruch 1, **dadurch gekennzeichnet, dass** es sich bei dem Implantatrohling um einen ungesinterten Rohling handelt.

3. Verfahren nach Anspruch 1 oder 2, **dadurch gekennzeichnet, dass** der Implantatrohling ein Prothesenrohling, vorzugsweise ein Gefäßprothesenrohling, ist.

4. Verfahren nach einem der vorhergehenden Ansprüche, **dadurch gekennzeichnet, dass** das antimikrobiell wirksame Metall ausgewählt ist aus der Gruppe bestehend aus Iridium, Aluminium, Zink, Kupfer, Silber, Gold, Titan, Zirkonium, Platin, Palladium und Kombinationen, insbesondere Legierungen, davon.

5. Verfahren nach einem der vorhergehenden Ansprüche, **dadurch gekennzeichnet, dass** der Implantatrohling mit Silber beschichtet wird.

6. Verfahren nach einem der vorhergehenden Ansprüche, **dadurch gekennzeichnet, dass** der beschichtete Implantatrohling auf das 0,5 bis 10-fache, insbesondere 1,5 bis 7-fache, vorzugsweise 3 bis 5-fache, seiner ursprünglichen Länge verstreckt wird.

7. Verfahren nach einem der vorhergehenden Ansprüche, **dadurch gekennzeichnet, dass** der beschichtete Implantatrohling nach dem Verstrecken gesintert wird, vorzugsweise oberhalb der Kristallisationstemperatur von Polytetrafluorethylen und/oder des Tetrafluorethylen als Monomereinheit umfassenden Copolymers.

8. Implantat mit einer Knoten-Fibrillen-Struktur, hergestellt oder herstellbar nach einem Verfahren nach einem der vorhergehenden Ansprüche.

9. Implantat nach Anspruch 8, **dadurch gekennzeichnet, dass** sämtliche Fibrillen der Knoten-Fibrillen-Struktur beidseitig mit den Knoten verbunden sind.

10. Implantat nach Anspruch 8 oder 9, **dadurch gekennzeichnet, dass** die Fibrillen der Knoten-Fibrillen-Struktur frei von einer Beschichtung mit dem antimikrobiell wirksamen Metall sind.

11. Implantat nach Anspruch 8 oder 9, **dadurch gekennzeichnet, dass** die Fibrillen an der Oberfläche, insbesondere Außenoberfläche, des Implantats wenigstens teilbeschichtet, vorzugsweise nur teilbeschichtet, vorliegen.

12. Implantat nach einem der Ansprüche 8 bis 11, **dadurch gekennzeichnet, dass** das Implantat einen Anteil an dem antimikrobiell wirksamen Metall zwischen 0,01 und 5,0 Gew.-%, insbesondere 0,05 und 3,0 Gew.-%, bevorzugt 0,1 und 1,5 Gew.-%, bezogen auf das Gesamtgewicht des Implantats aufweist.

13. Implantat nach einem der Ansprüche 8 bis 12, **dadurch gekennzeichnet, dass** die beschichteten Knoten eine Schichtdicke des antimikrobiell wirksamen Metalls zwischen 50 und 4000 Å, insbesondere 200 und 2000 Å, vorzugsweise 250 und 1500 Å, aufweisen.

14. Implantat nach einem der Ansprüche 9 bis 13, **dadurch gekennzeichnet, dass** die internodalen Zwischenräume der Knoten-Fibrillen-Struktur frei von einer Beschichtung mit dem antimikrobiell wirksamen Metall sind.

## Revendications

1. Procédé de production d'un implant revêtu, comprenant les étapes consistant à :
a) revêtir une préforme d'implant non étirée, comprenant du polytétrafluoroéthylène et/ou un copolymère comprenant du tétrafluoroéthylène comme unité monomère, par un métal à effet antimicrobien, le revêtement étant effectué au moyen d'un dépôt assisté par canon à ions (ion beam assisted deposition - IBAD) et
b) étirer la préforme d'implant revêtue.

2. Procédé selon la revendication 1, **caractérisé en ce que** la préforme d'implant est une préforme non frittée.

3. Procédé selon la revendication 1 ou 2, **caractérisé en ce que** la préforme d'implant est une préforme prothétique, de préférence une préforme prothétique vasculaire.

4. Procédé selon l'une quelconque des revendications précédentes, **caractérisé en ce que** le métal à effet antimicrobien est choisi dans le groupe constitué par l'iridium, l'aluminium, le zinc, le cuivre, l'argent, l'or, le titane, le zirconium, le platine, le palladium et leurs combinaisons, en particulier les alliages.

5. Procédé selon l'une quelconque des revendications précédentes, **caractérisé en ce que** la préforme d'implant est revêtue par de l'argent.

6. Procédé selon l'une quelconque des revendications précédentes, **caractérisé en ce que** la préforme d'implant revêtue est étirée 0,5 à 10 fois, en particulier 1,5 à 7 fois, de préférence 3 à 5 fois par rapport à sa longueur originale.

7. Procédé selon l'une quelconque des revendications précédentes, **caractérisé en ce que** la préforme d'implant revêtue est frittée après l'étirage, de préférence au-dessus de la température de cristallisation du polytétrafluoroéthylène et/ou du copolymère comprenant du tétrafluoroéthylène comme unité monomère.

8. Implant présentant une structure nodule-fibrilles produit ou pouvant être produit par un procédé selon l'une quelconque des revendications précédentes.

9. Implant selon la revendication 8, **caractérisé en ce que** toutes les fibrilles de la structure nodule-fibrilles sont reliées des deux côtés aux nodules.

10. Implant selon la revendication 8 ou 9, **caractérisé en ce que** les fibrilles de la structure nodule-fibrilles sont exemptes de revêtement présentant le métal à effet antimicrobien.

11. Implant selon la revendication 8 ou 9, **caractérisé en ce que** les fibrilles se trouvant à la surface de l'implant, en particulier à la surface externe, sont au moins partiellement revêtues et de préférence uniquement partiellement revêtues.

12. Implant selon l'une quelconque des revendications 8 à 11, **caractérisé en ce que** l'implant comprend une proportion du métal à effet antimicrobien entre 0,01 et 5,0% en poids, en particulier entre 0,05 et 3,0% en poids, de préférence entre 0,1 et 1,5% en poids, exprimée en termes de poids total de l'implant.

13. Implant selon l'une quelconque des revendications 8 à 12, **caractérisé en ce que** les nodules revêtus présentent une épaisseur de couche du métal à effet antimicrobien entre 50 et 4000 Å, en particulier entre 200 et 2000 Å, de préférence entre 250 et 1500 Å.

14. Implant selon l'une quelconque des revendications 9 à 13, **caractérisé en ce que** les espaces entre les nodules de la structure nodule-fibrilles sont exempts de revêtement présentant le métal à effet antimicrobien.
